# FASCICULE DE BREVET EUROPEEN

(11) **EP 1 032 565 B1**
(45) Date de publication et mention de la délivrance du brevet: **19.02.2003**
(21) Numéro de dépôt: 98955658.4
(22) Date de dépôt: 16.11.1998
(51) Int. Cl.: C07D 305/14

(54) **PROCEDE DE PREPARATION DE DERIVES DE LA CLASSE DES TAXOIDES**
VERFAHREN ZUR HERSTELLUNG VON DERIVATEN DER TAXOID-KLASSE
METHOD FOR PREPARING DERIVATIVES OF THE TAXOID CLASS

(30) Priorité: 18.11.1997 FR 9714442
(43) Date de publication de la demande: 06.09.2000
(73) Titulaire: Aventis Pharma S.A., 92160 Antony (FR)
(72) Inventeur: DIDIER, Eric, F-75013 Paris (FR); ODDON, Gilles, F-69003 Lyon (FR); PAUZE, Denis, F-69360 Solaize (FR); LEON, Patrick, F-69160 Tassin-la-Demi-Lune (FR); RIGUET, Didier, F-69360 Serezin du Rhône (FR)
(74) Mandataire: Le Pennec, Magali
(86) Numéro de dépôt international: FR9802432
(87) Numéro de publication internationale: WO99025704

(56) Documents cités:
- EP-A- 0 304 244
- EP-A- 0 400 971
- EP-A- 0 728 724
- WO-A-95/25728
- WO-A-95/29926
- WO-A-96/30355
- FR-A- 2 255 279
- CHEMICAL ABSTRACTS, vol. 111, no. 25, 18 décembre 1989 Columbus, Ohio, US; abstract no. 232028, XP002072628 -& J. DE LA ZERDA ET AL.: TETRAHEDRON, vol. 45, no. 5, 1989, pages 1533-1536, XP002072626 OXFORD GB
- CHEMICAL ABSTRACTS, vol. 128, no. 15, 13 avril 1998 Columbus, Ohio, US; abstract no. 180145, XP002072629 & M. ANTOSZCZYSZYN ET AL.: PRZEM. CHEM., vol. 76, no. 12, 1997, pages 525-527,
- CHEMICAL ABSTRACTS, vol. 121, no. 21, 21 novembre 1994 Columbus, Ohio, US; abstract no. 255527, XP002072630 & Z. TANG ET AL.: HUAXUE SHIJI, vol. 16, no. 2, 1994, pages 107-109,
- CHEMICAL ABSTRACTS, vol. 120, no. 21, 23 mai 1994 Columbus, Ohio, US; abstract no. 269678, XP002072631 & E.A. TRAUFFER: DISS. ABSTR. INT. B, vol. 53, no. 6, 1992, page 2880
- T. NISHIGUCHI ETA L.: JOURNAL OF THE CHEMICAL SOCIETY, PERKIN TRANSACTIONS 1, vol. 1, 1992, pages 153-156, XP002072627 LETCHWORTH GB

## Description

La présente invention concerne un nouveau procédé de préparation de dérivés dialkoxylés de la classe des taxoïdes. On entend par dérivés dialkoxylés de la classe des taxoïdes des dérivés porteurs en position 7 et 10 du noyau baccatine d'un motif alkoxy et éventuellement porteurs en position 13 d'une chaîne β-phénylisosérine.

On entend plus précisement par dérivés dialkoxylés de la classe des taxoïdes les dérivés répondant à la formule générale suivante : 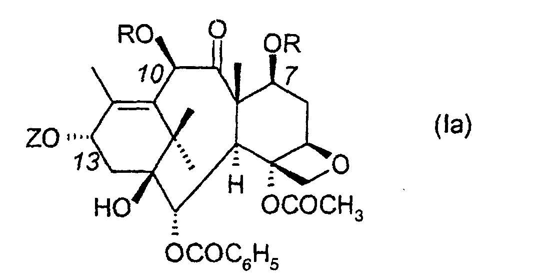 dans laquelle :
- les groupes R représentent un même groupe alkyle droit ou ramifié contenant 1 à 6 atomes de carbone
- Z représente l'hydrogène ou un motif de formule 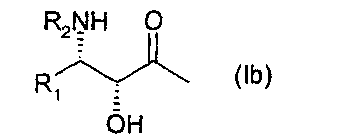
- dans lequel R₁ représente
   1 ) un radical alkyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalkyle contenant 3 à 6 atomes de carbone, phényle ou α- ou β-naphtyle éventuellement substitué par un ou plusieurs atomes ou radicaux choisis parmi les atomes d'halogène et les radicaux alkyles, alcényles, alcynyles, aryles, aralkyles, alcoxy, alkylthio, aryloxy, arylthio, hydroxy, hydroxyalkyle, mercapto, formyle, acyle, acylamino, aroylamino, alcoxycarbonyl-amino, amino, alkylamino, dialkylamino, carboxy, alcoxycarbonyle, carbamoyle, alkylcarbamoyle, dialkylcarbamoyle, cyano, nitro et trifluorométhyle, ou
   2) un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alkyles, aryles, amino, alkylamino, dialkylamino, alcoxycarbonylamino, acyle, arylcarbonyle, cyano, carboxy, carbamoyle, alkylcarbamoyle, dialkylcarbamoyle ou alcoxycarbonyle,
   3) étant entendu que, dans les substituants des radicaux phényle, α- ou β-naphtyle et hétérocyclyles aromatiques, les radicaux alkyles et les portions alkyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
- R₂ représente
   1) un radical benzoyle éventuellement substitué par un ou plusieurs atomes ou radicaux, identiques ou différents, choisis parmi les atomes d'halogène et les radicaux alkyles contenant 1 à 4 atomes de carbone, alcoxy contenant 1 à 4 atomes de carbone ou trifluorométhyle, thénoyle ou furoyle ou
   2) un radical R'₂-O-CO- dans lequel R'₂ représente :
- un radical alkyle contenant 1 à 8 atomes de carbone, alcényle contenant 2 à 8 atomes de carbone, alcynyle contenant 3 à 8 atomes de carbone, cycloalkyle contenant 3 à 6 atomes de carbone, cycloalcényle contenant 4 à 6 atomes de carbone, bicycloalkyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialkylamino dont chaque partie alkyle contient 1 à 4 atomes de carbone, pipéridino, morpholino, pipérazinyl-1 (éventuellement substitué en -4 par un radical alkyle contenant 1 à 4 atomes de carbone ou par un radical phénylalkyle dont la partie alkyle contient 1 à 4 atomes de carbone), cycloalkyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alkyles contenant 1 à 4 atomes de carbone ou alcoxy contenant 1 à 4 atomes de carbone), cyano, carboxy ou alcoxycarbonyle dont la partie alkyle contient 1 à 4 atomes de carbone,
- un radical phényle ou α- ou β-naphtyle éventuellement substitué par un ou plusieurs atomes ou radicaux choisis parmi les atomes d'halogène et les radicaux alkyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alkyles contenant 1 à 4 atomes de carbone.

Parmi les produits de formule (la) objet du procédé de la présente invention on préfère ceux pour lesquels :
- Z représente l'hydrogène ou un radical de formule (Ib) dans lequel
- R₁ représente un radical phényle
- R₂ représente un radical terbutoxycarbonyle ou un radical benzoyle.

On préfère tout particulièrement ceux pour lesquels:
- R₁ représente un radical phényle
- R₂ représente un radical terbutoxycarbonyle
- R représente un radical méthyle.

Il est connu selon le brevet WO96/30355 de préparer un dérivé selon la présente invention par deux procédés. Selon un premier procédé en plusieurs étapes, au départ de la 10-désacétyl-baccatine III de formule : 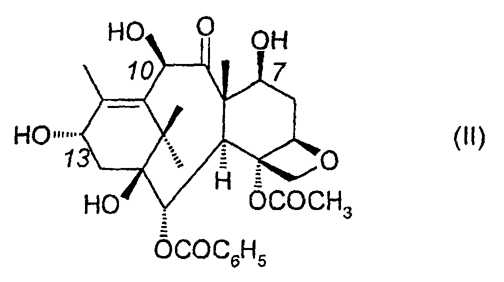 on la protége sélectivement en position 7 et 13, par exemple sous forme d'un diéther silylé, suivi de l'action d'un produit de formule générale :

R-X (III)

dans laquelle R représente un radical tel que défini précédemment et X représente un reste d'ester réactif tel qu'un reste d'ester sulfurique ou sulfonique ou un atome d'halogène pour obtenir un produit porteur du motif -OR en position 10 et de groupes silylés en position 7 et 13. Ensuite les groupes protecteurs silylés sont remplacés par des atomes d'hydrogène pour obtenir un composé toujours porteur en position 10 du groupe -OR et de groupes OH en position 7 et 13. Ce dernier dérivé est éthérifié en position 7 sélectivement par réaction avec le dérivé de formule (III) afin d'obtenir le dérivé de formule (I) dans lequel Z est égal à l'hydrogène.

La dernière étape consiste à estérifier en position 13, selon un procédé connu en soi, les dérivés de formule (la), dans lesquels Z réprésente l'hydrogène, en présence d'un β-lactame selon par exemple le procédé décrit dans le brevet EP 617018 ou en présence d'une oxazolidine selon par exemple le procédé décrit dans le brevet WO96/30355 précédemment cité.

Selon un deuxième procédé décrit dans le même brevet WO96/30355, les produits de formule générale (la) peuvent être obtenus selon un procédé en 5 étapes à partir du produit de formule (II). Dans une première étape on réalise une protection des positions 7 et 10 puis une estérification en position 13 en présence d'un β lactame selon par exemple le procédé décrit dans le brevet EP 617018 ou en présence d'une oxazolidine tel que par exemple décrit dans brevet WO96/30355 précédemment cité. Après déprotection des groupes protecteurs en position 7 et 10, on obtient ainsi un ester de formule (la) dans lequel Z est différent de l'hydrogène et R représente l'hydrogène. L'étape suivante consiste à faire réagir les positions 7 et 10 simultanément par l'action d'un réactif formé in situ à partir d'un sulfoxide de formule (IV) et d'anhydride acétique (réaction de type Pummerer),

R-SO-R (IV)

dans laquelle R a la même signification que précédemment pour former un intermédiaire de type alkylthioalkyloxy sur les positions 7 et 10.

La dernière étape qui permet d'obtenir le composé désiré de formule (la) est réalisée sur le composé intermédiaire obtenu précédemment par action de Nickel de Raney activé.

Généralement l'action du réactif formé in situ à partir du sulfoxyde de formule générale (IV), de préférence le diméthylsulfoxyde et l'anhydride acétique, s'effectue en présence d'acide acétique ou d'un dérivé de l'acide acétique tel qu'un acide halogénoacétique à une température comprise entre 0 et 50°C.

Généralement, l'action du nickel de Raney activé en présence d'un alcool aliphatique ou d'un éther est effectuée à une température comprise entre -10 et 60°C.

L'ensemble des procédés décrits dans cet art antérieur n'a jamais permis d'atteindre directement en une seule étape les dérivés dialcoxylés en position 7 et 10 de la 10-désacétylbaccatine III.

La présente invention a permis d'atteindre cet objectif. Elle permet en une seule étape l'alkylation directe, sélective et simultanée des deux fonctions hydroxyle en position 7 et 10 de la 10-désacétylbaccatine ou des dérivés de cette dernière estérifiés en position 13 de formule (V) 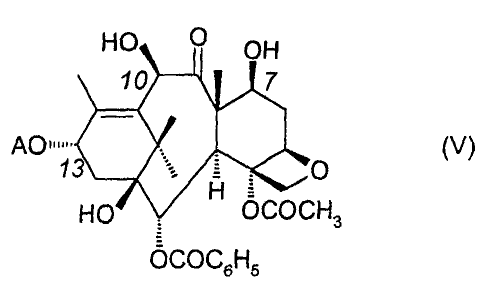 dans laquelle A représente l'hydrogène ou une chaîne latérale de formule (lc) suivante : 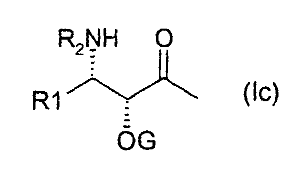 dans laquelle G représente un groupe protecteur de la fonction hydroxy
ou un motif oxazolidine de formule (Id): 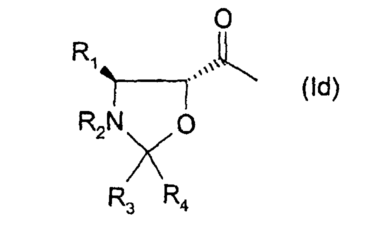 dans lesquelles R₁ et R₂ ont les mêmes significations que précédemment et R3 et R4 sont choisis parmi l'hydrogène ou parmi les radicaux alkyles, aryles, halogéno, alkoxy, arylalkyle, alkoxyaryle, halogenoalkyle, halogénoaryle, les substituants pouvant éventuellement former un cycle à 4 à 7 chaînons.

On préfère utiliser comme matière de départ la 10-désacétylbaccatine c'est à dire le produit de formule (II) ce qui permet une économie notable en ce qui concerne le procédé et évite d'autre part les étapes de protection et déprotection intermédiaires nécessaires dans les procédés de l'art antérieur.

Parmi les groupes G protecteurs de la fonction hydroxy de la formule (Ic), on préfère choisir de manière générale l'ensemble des groupements protecteurs décrits dans des ouvrages tels que Greene et Wuts Protective Groups in Organics Synthesis 1991, John Wileys & Sons et Mac Omie Protective Groups in Organic Chemistriy 1975 Plenum Press et qui se déprotègent dans des conditions qui dégradent peu ou pas le reste de la molécule, comme par exemple:
- les éthers et de préférence les éthers tels que le méthoxyméthyléther, le 1-éthoxyéthyléther, le benzyloxyméthyléther, le p-méthoxybenzyloxyméthyléther, les benzyléthers éventuellement substitués par un ou plusieurs groupes tels que méthoxy, chloro nitro, le 1-méthyl-1-méthoxyéthyléther, le 2-(triméthylsilyl)éthoxyméthyléther, le tétrahydro-pyranyléther, les éthers silylés tels que les trialkylsilyléthers,
- les carbonates tels que le trichloroéthylcarbonates.

Plus particulièrement, les radicaux R₃ et R₄ de la formule générale (Id) sont choisis parmi ceux décrits dans le brevet WO94/07878 et plus particulièrement on préfère les dérivés où R₃ est l'hydrogène et R₄ le radical p-méthoxyphényle.

L'agent d'alkylation est choisi parmi :
- les halogénures d'alkyle et de préférence parmi eux les iodures d'alkyle (RI)
- les sulfates d'alkyle tels que le sulfate de méthyle
- les oxoniums tels que les sels boriques de trialkyloxoniums notamment le triméthyloxonium tetrafluoroborate (Me₃OBF₄).

On utilise de préférence l'iodure de méthyle.

L'agent d'alkylation est utilisé en présence d'un agent d'anionisation tel qu'une ou plusieurs bases fortes en milieu anhydre.

Parmi les bases utilisables en milieu anhydre on peut citer :
- les hydrures alcalins tels que l'hydrure de sodium ou de potassium
- les alcoolates alcalins tels que le terbutylate de potassium
- l'oxyde d'argent Ag₂O
- le 1,8-bis(diméthylamino)naphthalène
- les mélanges de base uni ou bimétalliques tels que ceux par exemple décrits dans des publications telles que P. Caubère Chem. Rev. 1993, 93, 2317-2334 ou M. Schlosser Mod. Synth. Methods (1992), 6, 227-271, en particulier les associations alkyllithium/ t-butylate alcalin ou amidures alcalin/t-butylate alcalin sont préférées. Une des deux bases peut être générée "in situ".

Parmi toutes les combinaisons possibles agent d'alkylation et agent d'anionisation, on préfère utiliser l'iodure de méthyle en présence d'hydrure de potassium.

De préférence la réaction est réalisée dans un milieu organique inerte dans les conditions de la réaction. On préfère parmi les solvants utiliser :
- les éthers tels que le tétrahydrofurane, le diméthoxyéthane
- lorsque l'on utilise l'oxyde d'argent on préfère utiliser les solvants aprotiques polaires tels que le diméthylformamide ou les solvants aromatiques tels que le toluène
- lorsque l'on utilise le 1,8-bis(diméthylamino)naphthalène, on préfère utiliser les esters tels que l'acétate d'éthyle.

Pour une meilleure mise en oeuvre de l'invention on préfère utiliser un rapport molaire entre l'agent d'anionisation et le substrat supérieur à 2 et de préférence compris entre 2 et 20.

On préfère aussi utiliser un rapport molaire entre l'agent d'alkylation et le substrat supérieur à 2 et de préférence compris entre 2 et 40.

On préfère utiliser une température réactionnelle comprise entre -30°C et 80°C.

La durée de réaction varie avantageusement entre quelques heures et 48 heures suivant les réactifs choisis.

Après l'étape d'alkylation, lorsque cette dernière est réalisée sur la 10-désacétylbaccatine, on procède de manière connue à l'étape d'estérification selon, par exemple, les procédés décrits dans les brevets EP 617018 ou WO96/30355 précédemment cités.

Ainsi selon un premier procédé en 3 étapes on procède à partir de la 10-désacétylbaccatine tout d'abord à la dialkylation par utilisation d'un agent alkylant en présence d'une base forte, dans une deuxième étape on condense en position 13 la 10-désacétylbaccatine diéthérifée en position 7 et 10 avec un β-lactame convenablement protégé en présence d'un agent d'activation choisi parmi les amines tertiaires et les bases métalliques assurant la formation d'un alcoolate en position 13. La déprotection de la chaîne latérale est assurée ensuite par l'action d'un acide minéral ou organique.

Ainsi selon un deuxième procédé en 3 étapes on procède à partir de la 10-désacétylbaccatine tout d'abord à la dialkylation par utilisation d'un agent alkylant en présence d'une base forte, dans une deuxième étape on condense en position 13 de la 10-désacétylbaccatine diéthérifée en position 7 et 10 une oxazolidine en présence d'un agent de couplage tel que les diimides en présence d'un agent activant tel que les dialkylaminopyridines. L'ouverture de l'oxazolidine est assurée par l'action d'un acide minéral ou organique.

Selon un troisième procédé, on procède d'abord à l'estérification en position 13 de la baccatine convenablement protégée en positon 7 et 10 avec un β-lactame ou une oxazolidine en présence d'un agent de couplage et ou d'activation comme décrit dans les deux procédés précédents. Après déprotection en position 7 et 10, la diéthérification en position 7 et 10 est effectuée par un agent alkylant en présence d'une base forte. La déprotection de la chaîne latérale est assurée ensuite par l'action d'un acide minéral ou organique.

La présente invention sera plus complètement décrite à l'aide des exemples suivants qui ne doivent pas être considérés comme limitatifs de l'invention.

### EXEMPLES

Tous ces essais sont réalisés sous argon et en utilisant des solvants anhydres.

### Exemple 1 : oxyde d'argent / iodure de méthyle / toluène / 10-DAB

A une suspension de 10-désacétylbaccatine III (272 mg, 0,5 mmol) dans un mélange toluène/iodure de méthyle (3/2 ; 2,5 ml) à 0°C est ajouté l'oxyde d'argent (255 mg, 1,1 mmol, 2,2 équiv.). On laisse progressivement remonter la température à l'ambiante. Après 5h de réaction, le mélange réactionnel est chauffé à 60°C. Après 24h d'agitation à 60°C est rajouté un excès de réactifs : oxyde d'argent (2x255 mg) et iodure de méthyle (2x1 ml). Après 36h supplémentaires de chauffage, le mélange réactionnel est filtré sur verre fritté et le filtrat est évaporé. D'après l'analyse HPLC, le milieu réactionnel contient 11,5 % (en normalisation interne des surfaces) de 7,10-diméthoxy-10-désacétylbaccatine.

### Exemple 2 : oxyde d'argent / iodure de méthyle / pyridine / toluène / 10-DAB

A une suspension de 10-désacétylbaccatine III (272 mg, 0,5 mmol) dans un mélange toluène/iodure de méthyle (3/2 ; 2,5 ml) à température ambiante sont successivement ajoutés la pyridine (8 µl, 0,1 mmol, 0,2 équiv.) puis l'oxyde d'argent (255 mg, 1,1 mmol, 2,2 équiv.). Le mélange réactionnel est ensuite chauffé à 50°C. Après 24h d'agitation à 60°C est rajouté un excès de réactifs : oxyde d'argent (255 mg, 1,1 mmol, 2,2 équiv.), pyridine (80 µl, 1 mmol, 2 équiv.) et iodure de méthyle (1 ml). Après 24h supplémentaires de chauffage , le mélange réactionnel est filtré sur verre fritté et le filtrat est dilué avec de l'acétate d'éthyle (40 ml). Cette phase est lavée à la saumure (20 ml), séparée, séchée sur sulfate de sodium et évaporée (131 mg) D'après l'analyse HPLC, le brut réactionnel contient 12,2 % (en normalisation interne des surfaces) de 7,10-diméthoxy-10-désacétylbaccatine.

### Exemple 3 : oxyde d'argent / iodure de méthyle / N,N-diméthylformamide / 10-DAB

A une solution de 10-désacétylbaccatine III (272 mg. 0,5 mmol) dans un mélange N,N-diméthylformamide/iodure de méthyle (3/2 ; 2,5 ml) à 0°C est ajouté l'oxyde d'argent (255 mg, 1,1 mmol, 2,2 équiv.). On laisse progressivement remonter la température à l'ambiante. Après 24h d'agitation, le mélange réactionnel est dilué au diéthyl éther (20 ml) et filtré sur verre fritté. Le filtrat est lavé à l'eau (20 ml). La phase organique est séparée, séchée sur sulfate de sodium et évaporée (207 mg). D'après l'analyse HPLC, le brut réactionnel contient 9,2 % (en normalisation interne des surfaces) de 7,10-diméthoxy-10-désacétylbaccatine.

### Exemple 4 : hydrure de potassium / iodure de méthyle / tétrahydrofurane / 10-DAB

L'hydrure de potassium en suspension à 20 % dans une huile minérale (6,0 g, 30 mmol, 3 équiv.) est préalablement lavé avec du pentane.

A une suspension d'hydrure de potassium, préalablement lavé au pentane, dans du tétrahydrofurane (30 ml) à -30°C est ajoutée au goutte à goutte une suspension de 10-désacétylbaccatine III (5,23 g, 8,5 mmol, pureté 89 %) dans un mélange tétrahydrofurane/iodure de méthyle (3/2, 50 ml). On laisse ensuite progressivemént remonter la température à l'ambiante. Après 3h30 d'agitation, le mélange réactionnel est versé sur de l'eau (150 ml) et du diisopropyléther (250 ml). Le mélange est filtré sur verre fritté. Le précipité est alors recueilli et lavé séparément avec de l'eau (14 ml). Cette suspension est à nouveau filtrée sur verre fritté pour fournir, après une nuit de séchage au dessiccateur sur P₂O₅, 3,17 g de 7,10-diméthoxy-10-désacétylbaccatine (pureté HPLC : 93 % en normalisation interne des surfaces). Le rendement en produit isolé est de 61 %.

### Exemple 5 : t-butylate de potassium / iodure de méthyle / tétrahydrofurane / 10-DAB

A une suspension de t-butylate de potassium (336 mg, 3 mmol, 3 équiv.) dans du tétrahydrofurane (4 ml) à -30°C est ajoutée au goutte à goutte une suspension de 10-désacétylbaccatine III (544 mg, 1 mmol) dans un mélange tétrahydrofurane/iodure de méthyle (3/2, 5 ml). On laisse ensuite progressivement remonter la température à l'ambiante. Après 3h30 d'agitation, l'analyse HPLC indique que le milieu réactionnel contient 10,0 % (en normalisation interne des surfaces) de 7,10-diméthoxy-10-désacétylbaccatine.

### Exemple 6 : hydrure de potassium / sulfate de méthyle / tétrahydrofurane / 10-DAB

L'hydrure de potassium en suspension à 20 % dans une huile minérale (0,6 g, 3 mmol, 3 équiv.) est préalablement lavé avec du pentane.

A une suspension d'hydrure de potassium, préalablement lavé au pentane, dans du tétrahydrofurane (3 ml) à -20°C sont ajoutées, simultanément et au goutte à goutte, une suspension de 10-désacétylbaccatine III (544 mg, 1 mmol) dans du tétrahydrofurane (6 ml) et une solution de sulfate de méthyle (2,0 g, 16 mmol, 16 équiv.) dans du tétrahydrofurane (2 ml). On laisse ensuite progressivement remonter la température à l'ambiante. Après 8h de réaction, le mélange réactionnel est versé sur de l'eau (20 ml) et placé une nuit à 4°C. On rajoute ensuite du diisopropyléther (20 ml) et le mélange est filtré sur verre fritté pour fournir 220 mg. D'après l'analyse CLHP, le produit brut contient 98 % (en normalisation interne des surfaces) de 7,10-diméthoxy-10-désacétylbaccatine.

### Exemple 7 : hydrure de potassium / tétrafluoroborate de triméthyloxonium / tétrahydrofurane / 10-DAB

L'hydrure de potassium en suspension à 20 % dans une huile minérale (0,6 g, 3 mmol, 3 équiv.) est préalablement lavé avec du pentane.

A une suspension d'hydrure de potassium, préalablement lavé au pentane, et de tétrafluoroborate de triméthyloxonium dans du tétrahydrofurane (3 ml) à -20°C est ajoutée une suspension de 10-désacétylbaccatine III (544 mg, 1 mmol) dans du tétrahydrofurane (3 ml). On laisse ensuite progressivement remonter la température à -10°C. Après deux heures de réaction est rajoutée une suspension d'hydrure de potassium (2 équiv.) dans du tétrahydrofurane (1 ml). Après deux heures supplémentaires de réaction, l'analyse HPLC indique que le mélange réactionnel contient 16,3 % (en normalisation interne des surfaces) de 7,10-diméthoxy-10-désacétylbaccatine.

### Exemple 8 : hydrure de potassium / iodure de méthyle / 1,2-diméthoxyéthane / 10-DAB

L'hydrure de potassium en suspension à 20 % dans une huile minérale (0,6 g, 3 mmol, 3 équiv.) est préalablement lavé avec du pentane.

A une suspension d'hydrure de potassium, préalablement lavé au pentane, dans du 1,2-diméthoxyéthane (3 ml) à -20°C est ajoutée au goutte à goutte une solution de 10-désacétylbaccatine III (544 mg, 1 mmol) dans un mélange 1,2-diméthoxyéthane/iodure de méthyle (3/1, 8 ml). On laisse ensuite progressivement remonter la température à l'ambiante. Après 6h30 d'agitation, l'analyse HPLC indique que le mélange réactionnel contient 28,1 % (en normalisation interne des surfaces) de 7,10-diméthoxy-10-désacétylbaccatine.

### Exemple 9 : Hydrure de potassium / iodure de méthyle / tétrahydrofurane / composé (V) avec A = (id) (R₁ = phényl, R₂=H, R₃= t-butoxycarbonyl, R₄= 4-méthoxyphényl

L'hydrure de potassium en suspension à 20 % dans une huile minérale (0,145 g, 2,4 équiv.) est préalablement lavé avec du pentane.

A une suspension d'hydrure de potassium, préalablement lavé au pentane, dans du tétrahydrofurane (0,7 ml) à -78°C est ajoutée au goutte à goutte une suspension de (2R,4S,5R)-3-tert-butoxycarbonyl-2-(4-méthoxyphényl)-4-phényl-5-oxazolidinecarboxylate de 4-acétoxy-2α-benzoyloxy-5β,20-époxy-1, 7β,10β-trihydroxy-9-oxo-tax-11-én-13α-yle (284 mg, 0,3 mmol) dans un mélange tétrahydrofurane/iodure de méthyle (5/3, 1,6 ml). On laisse ensuite progressivement remonter la température à -15°C. Après 3h30 d'agitation, le mélange réactionnel est versé sur de l'eau (15 ml) et de l'acétate d'éthyle (15 ml). La phase organique est séparée, lavée à la saumure (15 ml), séchée sur sulfate de sodium, filtrée et évaporée (232 mg). L'analyse HPLC du brut fournit un rendement dosé de 39 % en (2R,4S,5R)-3-tert-butoxycarbonyl-2-(4-méthoxyphényl)-4-phényl-5-oxazolidinecarboxylate de 4-acétoxy-2α-benzoyl-oxy-5β,20-époxy-1-hydroxy-9-oxo-7β,10β-dim thoxy-tax-11-én-13α-yle.

### Exemple 10 : hydrure de sodium / iodure de méthyle / tétrahydrofurane / 10-DAB

L'hydrure de sodium en suspension à 55 % dans une huile minérale (0,13 g, 3 mmol, 3 équiv.) est préalablement lavé avec du pentane.

A une suspension d'hydrure de sodium, préalablement lavé au pentane, dans du tétrahydrofurane (3 ml) à 0°C est ajoutée au goutte à goutte une suspension de 10-désacétylbaccatine III (544 mg, 1 mmol) dans un mélange tétrahydrofurane/iodure de méthyle (3/2, 5 ml). On laisse ensuite progressivement remonter la température à l'ambiante. Après 7h30 d'agitation, le mélange réactionnel est versé sur de l'eau (25 ml) et du diisopropyl éther (25 ml). Il apparaît un précipité qui est filtré sur verre fritté. On récupère ainsi 57 mg de produit contenant 67 % (en normalisation interne des surfaces) de 7,10-diméthoxy-10-désacétylbaccatine.

### Exemple 11 : n-butyllithium / t-butylate de potassium / tétrahydrofurane / 10-DAB

Une solution de n-butyllithium dans l'hexane (2 ml, 3 mmol, 3 équiv.) est évaporée sous vide. Le résidu est repris par du tétrahydrofurane (3 ml), préalablement refroidi à -78°C. On ajoute ensuite le t-butylate de potassium (336 mg, 3 mmol, 3 équiv.) puis on additionne une suspension de 10-désacétylbaccatine III (544 mg, 1 mmol) dans un mélange tétrahydrofuranne/iodure de méthyle (5 ml, 3/2). On laisse progressivement remonter la température à l'ambiante. Après 3h45 de réaction, le mélange est versé sur de l'eau (10 ml) et du diisopropyléther (10 ml). Après une nuit de cristallisation à 4°C, on récupère 75 mg de cristaux contenant 61 % (en normalisation interne des surfaces) de 7,10-diméthoxy-10-désacétylbaccatine.

### Exemple 12 : n-butyllithium / t-butylate de potasium / diisopropylamine / tétrahydrofurane / 10-DAB

Une solution de n-butyllithium dans l'hexane (2 ml, 3 mmol, 3 équiv.) est évaporée sous vide. Le résidu est repris par une solution de diisopropylamine (0,5 ml, 3 mmol, 3 équiv.) dans du tétrahydrofurane (3 ml), préalablement refroidie à -78°C. On ajoute ensuite le t-butylate de potassium (336 mg, 3 mmol, 3 équiv.) puis on additionne une suspension de 10-désacétylbaccatine III (544 mg, 1 mmol) dans un mélange tétrahydrofurane/iodure de méthyle (5 ml, 3/2). On laisse progressivement remonter la température à l'ambiante. Après 19h de réaction, l'analyse HPLC indique que le mélange réactionnel contient 24 % (en normalisation interne des surfaces) de 7,10-diméthoxy-10-désacétylbaccatine.

### Exemple 13 : amidure de sodium / t-butanol / tétrahydrofurane / 10-DAB

Une suspension d'amidure de sodium (173 mg, 4 mmol, 4 équiv.) et de t-butanol (0,13 ml, 1,3 mmol, 1,3 équiv.) dans du tétrahydrofurane (2 ml) est chauffée 2h à 45°C. Après retour à température ambiante, le mélange est refroidi à -50°C et une suspension de 10-désacétylbaccatine III (544 mg, 1 mmol) dans un mélange tétrahydrofurane/iodure de méthyle (3/2, 5 ml) est additionnée au goutte à goutte. On laisse progressivement remonter la température à -20°C. Après 2h20 d'agitation, le mélange est versé sur de l'eau (10 ml) et du diisopropyléther (10 ml). Le précipité est filtré sur verre fritté pour fournir 160 mg de brut contenant 37 % (en normalisation interne des surfaces) de 7,10-diméthoxy-10-désacétylbaccatine.

### Exemple 14: tétrafluoroborate de triméthyloxonium / le 1,8-bis(diméthylamino)naphthalène / tamis 4Å / 10-DAB

A une suspension de 10-désacétylbaccatine III (109 mg, 0,2 mmol) dans du dichlorométhane (4 ml) à 25°C sont successivement ajoutés le 1,8-bis(diméthylamino)naphthalène (514 mg, 2,4 mmol, 12 équiv.), du tamis moléculaire 4Å (700 mg) et du tétrafluoroborate de triméthyloxonium (296 mg, 2 mmol, 10 équiv.). Après 24h d'agitation à température ambiante, l'analyse HPLC indique que le mélange réactionnel contient la 7,10-diméthoxy-10-désacétylbaccatine avec un rendement dosé de 17 %.

### Exemple 15

A une suspension de 10-désacétylbaccatine III (0,2876 g, 0,46 mmol) dans de l'acétate d'éthyle (7,8 ml) à 20°C sont successivement ajoutés le 1,8 bis(diméthylamino)naphtalène (1,2744 g, 5,95 mmol, 12,8 équiv) et du tétrafluoroborate de triméthyloxonium (0,7598 g, 14 mmol, 11 équiv). Après 2 heures 20 minutes d'agition à une température comprise entre 45-50°C, l'analyse HPLC indique que le mélange réactionnel contient la 7,10-diméthoxy-10-désacétylbaccatine avec un rendement dosé de 62 %.

### Analyses pour le composé 7,10-diméthoxy-10-désacétylbaccatine III

Les analyses RMN ont été effectuées sur un spectromètre Bruker AM 360 opérant à 360 MHz pour le proton et 90 MHz pour le carbone 13 et équipé d'une sonde duale proton/carbone 13 de 5 mm. Les déplacements chimiques sont exprimés en ppm ; le DMSO est utilisé comme référence externe (2,44 ppm en proton et 39,5 ppm en carbone). La température est contrôlée par une unité de température variable à 300 K.

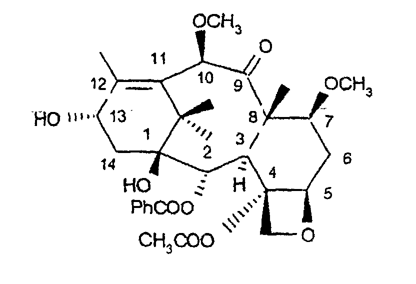

| Position | RMN ¹H | | | RMN ¹³C |
|---|---|---|---|---|
| | δ (ppm) | multiplicité | J (Hz) | d (ppm) |
| 1 | - | - | - | 75,2 |
| 2 | 5,34 | doublet | 7,2 | 74,3 |
| 3 | 3,71 | doublet | 7,2 | 47,0 |
| 4 | - | - | - | 80,0 |
| 5 | 4,93 | doublet | 9,1 | 83,2 |
| 6 | 1,44 / 2,64 | multiplet | - | 31,7 |
| 7 | 3,76 | doublet de doublet | 10,5 / 6,5 | 80,4 |
| 8 | - | - | - | 56,6 |
| 9 | - | - | - | 205,3 |
| 10 | 4,70 | singulet | - | 82,7 |
| 11 | - | - | - | 132,7 |
| 12 | - | - | - | 143,9 |
| 13 | 4,61 | multiplet | - | 66,1 |
| 14 | 2,13 | multiplet | - | 39,3 |
| 15 (CH₃) | 0,90 | singulet | - | 20,5 |
| | | | | 26,9 |
| 1 (OH) | 4,33 | singulet | - | - |
| 2 (PhCOO) | 7,5 (2H) | triplet | | 165,1 (C=O) |
| | 7,6 (1H) | triplet | | 130,1/129,4 |
| | 8,0 (ortho C=O) | doublet | 7,5 | 128,6/133,1 |
| 4 (Ac) | 2,15 | singulet | - | 169,6 (C=O) |
| | | | | 22,3 |
| 4 (CH₂O) | 3,99 | système AB | non déterminé | 76,8 |
| 7 (OCH₃) | 3,17 | singulet | - | 56,4 |
| 8 (CH₃) | 1,47 | singulet | - | 10,0 |
| 10 (OCH₃) | 3,25 | singulet | - | 56,0 |
| 12 (CH₃) | 1,93 | singulet | - | 15,0 |
| 13 (OH) | 5,25 | doublet | 4.5 | - |

SM : Introduction directe ; mode d'ionisation ESI+.

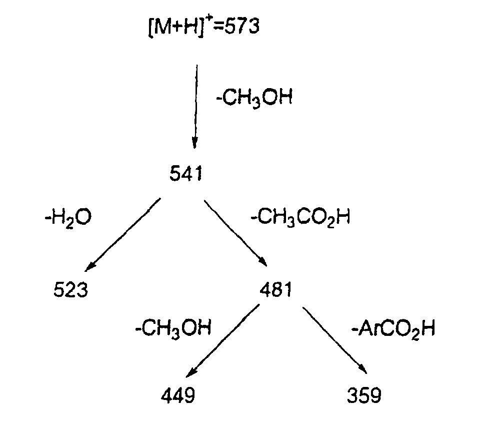

IR (KBr) :

| | |
|---|---|
| 3552,5 cm-1 | O-H alcoolique (secondaire) |
| 3434,9 cm-1 | O-H alcoolique (tertiaire) |
| 2972,2-2931,5-2892,6 cm-1 | squelette hydrocarboné |
| 2826,9 cm-1 | C-H des motifs O-CH3 |
| 1716-1705,3 cm-1 | C=O des motifs OAc et cétone |
| 1269,7-1250,8 cm-1 | C-O éther aromatique |
| 1098,3-1068,7 cm-1 | C-O des alcools et éther cyclique |

## Revendications

1. Procédé d'alkylation directe, en une seule étape, en position 7 et 10 simultanément des deux fonctions hydroxyles de la 10-désacétylbaccatine ou des dérivés de cette dernière estérifiés en position 13 de formule (V) 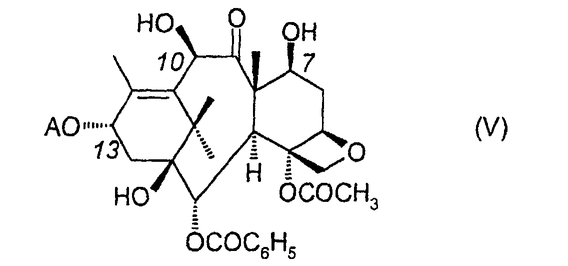 dans laquelle A représente l'hydrogène ou une chaîne latérale de formule (Ic) suivante : 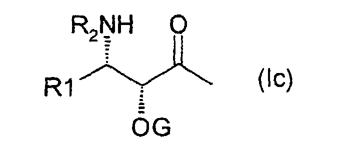 dans laquelle :
• G représente un groupe protecteur de la fonction hydroxyle,
• R₁ représente
1) un radical alkyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalkyle contenant 3 à 6 atomes de carbone, phényle ou α- ou β-naphtyle éventuellement substitué par un ou plusieurs atomes ou radicaux choisis parmi les atomes d'halogène et les radicaux alkyles, alcényles, alcynyles, aryles, aralkyles, alcoxy, alkylthio, aryloxy, arylthio, hydroxy, hydroxyalkyle, mercapto, formyle, acyle, acylamino, aroylamino, alcoxycarbonyl-amino, amino, alkylamino, dialkylamino, carboxy, alcoxycarbonyle, carbamoyle, alkylcarbamoyle, dialkylcarbamoyle, cyano, nitro et trifluorométhyle, ou
2) un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alkyles, aryles, amino, alkylamino, dialkylamino, alcoxycarbonylamino, acyle, arylcarbonyle, cyano, carboxy, carbamoyle, alkylcarbamoyle, dialkylcarbamoyle ou alcoxycarbonyle,
3) étant entendu que, dans les substituants des radicaux phényle, α-ou β-naphtyle et hétérocyclyles aromatiques, les radicaux alkyles et les portions alkyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles,
• R₂ représente
1) un radical benzoyle éventuellement substitué par un ou plusieurs atomes ou radicaux, identiques ou différents. choisis parmi les atomes d'halogène et les radicaux alkyles contenant 1 à 4 atomes de carbone, alcoxy contenant 1 à 4 atomes de carbone ou trifluorométhyle, thénoyle ou furoyle ou
2) un radical R'₂-O-CO- dans lequel R'₂ représente :
- un radical alkyle contenant 1 à 8 atomes de carbone, alcényle contenant 2 à 8 atomes de carbone, alcynyle contenant 3 à 8 atomes de carbone, cycloalkyle contenant 3 à 6 atomes de carbone, cycloalcényle contenant 4 à 6 atomes de carbone, bicycloalkyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialkylamino dont chaque partie alkyle contient 1 à 4 atomes de carbone, pipéridino, morpholino, pipérazinyl-1 (éventuellement substitué en -4 par un radical alkyle contenant 1 à 4 atomes de carbone ou par un radical phénylalkyle dont la partie alkyle contient 1 à 4 atomes de carbone), cycloalkyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alkyles contenant 1 à 4 atomes de carbone ou alcoxy contenant 1 à 4 atomes de carbone), cyano, carboxy ou alcoxycarbonyle dont la partie alkyle contient 1 à 4 atomes de carbone,
- un radical phényle ou α- ou β-naphtyle éventuellement substitué par un ou plusieurs atomes ou radicaux choisis parmi les atomes d'halogène et les radicaux alkyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
ou un radical hétérocyclyle saturé contenant 4 à 6 atomes de carbone éventuellement substitué par un ou plusieurs radicaux alkyles contenant 1 à 4 atomes de carbone,
ou une oxazolidine de formule(ld) suivante : 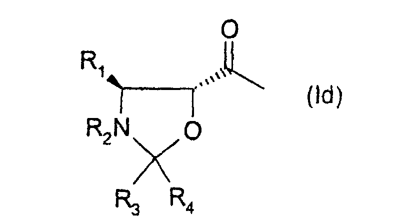 dans laquelle R₃ et R₄ sont choisis parmi l'hydrogène ou les radicaux alkyles, aryles, halogéno, alkoxy, arylalkyle, alkoxyaryle, halogenoalkyle, halogénoaryle, les substituants pouvant éventuellement former un cycle à 4 à 7 chaînons,
**caractérisé en ce que** l'agent d'alkylation est choisi parmi :
• les halogénures d'alkyle et de préférence parmi eux les iodures d'alkyle (RI)
• les sulfates d'alkyle tels que le sulfate de méthyle
• les oxoniums tels que les sels boriques de trialkyloxoniums notamment le triméthyloxonium tétrafluoroborate
en présence d'un ou plusieurs agents d'anionisation tel qu'une ou plusieurs bases fortes en milieu anhydre.

2. Procédé selon la revendication 1 caractérisé en que l'agent d'alkylation est le sulfate ou l'iodure de méthyle et de préférence l'iodure de méthyle.

3. Procédé selon la revendication 1 caractérisé en que les bases utilisables en milieu anhydre sont choisis parmi les hydrures alcalins, les alcoolates alcalins, l'oxyde d'argent, le 1,8-bis(diméthylamino)naphthalène, les amidures alcalins en mélange avec le t-butylates alcalins ou les alkyllithiums en mélange avec les t-butylates alcalins.

4. Procédé selon la revendication 3 **caractérisé en ce que** les hydrures alcalins sont choisis parmi l'hydrure de sodium ou de potassium et de préférence est l'hydrure de potassium.

5. Procédé selon la revendication 3 **caractérisé en ce que** l'alcoolate alcalin est le terbutylate de potassium.

6. Procédé selon la revendication 1 caractérisé en que l'agent d'alkylation est l'iodure de méthyle en présence d'hydrure de potassium.

7. Procédé selon l'une quelconque des revendications précédentes caractérisé en de que la réaction est réalisée dans un milieu organique inerte dans les conditions de la réaction.

8. Procédé selon les revendications 4 à 6 **caractérisé en ce que** l'on utilise un solvant choisi parmi les éthers.

9. Procédé selon la revendication 3 **caractérisé en ce que** lorsque la réaction est réalisée en présence d'oxyde d'argent, le solvant utilisé est choisi parmi les solvants aromatiques et les solvants aprotiques polaires.

10. Procédé selon la revendication 8 **caractérisé en ce que** l'on utilise comme solvant le tétrahydrofurane ou le diméthoxyéthane.

11. Procédé selon les revendications 1 et 3 **caractérisé en ce que** l'on utilise un mélange 1,8 bis(diméthylamino)naphtalène, tétrafluoroborate de triméthyloxonium dans l'acétate d'éthyle.

12. Procédé selon l'une quelconque des revendications précédentes **caractérisé en ce que** l'on utilise un rapport molaire entre l'agent d'anionisation et le substrat supérieur à 2 et de préférence compris entre 2 et 20.

13. Procédé selon l'une quelconque des revendications précédentes **caractérisé en ce que** l'on utilise un rapport molaire entre l'agent alkylant et le substrat supérieur à 2 et de préférence compris entre 2 et 40.

14. Procédé selon l'une quelconque des revendications précédentes caractérisé en qu'on utilise une température réactionnelle comprise entre -30°C et 80°C.

## Patentansprüche

1. Verfahren zur direkten Alkylierung in einer einzigen Stufe von gleichzeitig zwei Hydroxylfunktionen in Position 7 und 10 von 10-Desacetyl-Baccatin oder dessen in Position 13 veresterten Derivaten der Formel (V) 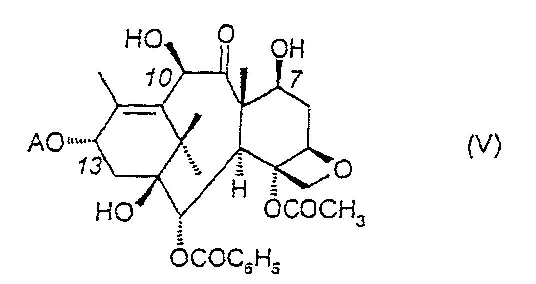 in der A Wasserstoff oder eine Seitenkette der folgenden Formel (Ic) 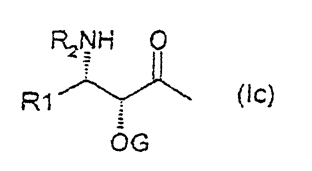 darstellt, worin
• G eine Schutzgruppe für die Hydroxylfunktion ist,
• R₁ bedeutet:
1) einen geraden oder verzweigten Rest Alkyl mit 1 bis 8 Kohlenstoffatomen, einen geraden oder verzweigten Rest Alkenyl mit 2 bis 8 Kohlenstoffatomen, einen geraden oder verzweigten Rest Alkinyl mit 2 bis 8 Kohlenstoffatomen, Cycloalkyl mit 3 bis 6 Kohlenstoffatomen, Phenyl oder α- oder β-Naphthyl, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder
2) einen aromatischen Heterocyclus mit 5 Ringgliedern und einem oder mehreren, gleichen oder verschiedenen Heteroatomen, ausgewählt unter den Atomen von Stickstoff, Sauerstoff oder Schwefel und gegebenenfalls substituiert durch einen oder mehrere, gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl,
3) mit der Maßgabe, daß in den Substituenten der Reste Phenyl, α- oder β-Naphthyl und aromatischem Heterocyclyl die Reste Alkyl und die Teile Alkyl der anderen Reste 1 bis 4 Kohlenstoffatome enthalten und daß die Reste Alkenyl und Alkinyl 2 bis 8 Kohlenstoffatome enthalten und daß die Reste Aryl Reste Phenyl oder α- oder β-Naphthyl sind,
• R₂ bedeutet:
1) einen Rest Benzoyl, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder Trifluormethyl, Thenoyl oder Furoyl, oder
2) einen Rest R'₂-O-CO-, worin R'₂ darstellt:
- einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkoxy mit 1 bis 4 Kohlenstoffatomen, Dialkylamino, bei dem jeder Teil Alkyl 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Teil Alkyl 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen), Cyano, Carboxy oder Alkoxycarbonyl, dessen Teil Alkyl 1 bis 4 Kohlenstoffatome enthält,
- einen Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen oder einem aromatischen heterocyclischen Rest mit 5 Ringgliedern, vorzugsweise ausgewählt unter den Resten Furyl und Thienyl, oder einen gesättigten heterocyclischen Rest mit 4 bis 6 Kohlenstoffatomen, gegebenenfalls substituiert durch einen oder mehrere Reste Alkyl mit 1 bis 4 Kohlenstoffatomen, oder
ein Oxazolidin der folgenden Formel (Id) 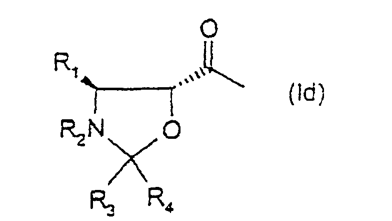 in der R₃ und R₄ ausgewählt werden unter Wasserstoff oder den Resten Alkyl, Aryl, Halogen, Alkoxy, Arylalkyl, Alkoxyaryl, Halogenalkyl, Halogenaryl, wobei die Substituenten gegebenenfalls einen Ring mit 4 bis 7 Ringgliedern bilden können,
**dadurch gekennzeichnet, daß** das Alkylierungsmittel ausgewählt wird unter:
• den Alkylhalogeniden und vorzugsweise unter diesen die Alkyliodide (RI),
• den Alkylsulfaten wie Methylsulfat,
• den Oxonium-Verbindungen wie den Borsalzen von Trialkyloxonium, insbesondere Trimethyloxonium-tetrafluorborat,
in Anwesenheit von einem oder mehreren Anionisierungsmitteln wie von einer oder mehreren starken Basen im wasserfreien Medium.

2. Verfahren nach Anspruch 1, **dadurch gekennzeichnet, daß** das Alkylierungsmittel Methylsulfat oder Methyliodid und vorzugsweise Methyliodid ist.

3. Verfahren nach Anspruch 1, **dadurch gekennzeichnet, daß** die im wasserfreien Medium verwendbaren Basen ausgewählt werden unter den Alkalihydriden, den Alkalialkoholaten, Silberoxid, 1,8-Bis-(Dimethylamino)-naphthalin, den Alkaliamiden in Mischung mit Alkali-tert.-Butylaten oder Alkyllithium in Mischung mit Alkalitert.-Butylaten.

4. Verfahren nach Anspruch 3, **dadurch gekennzeichnet, daß** die Alkalihydride ausgewählt werden unter Natriumhydrid oder Kaliumhydrid, und vorzugsweise ist es Kaliumhydrid.

5. Verfahren nach Anspruch 3, **dadurch gekennzeichnet, daß** das Alkalialkoholat das Kalium-tert.-butylat ist.

6. Verfahren nach Anspruch 1, **dadurch gekennzeichnet, daß** das Alkylierungsmittel Methyliodid in Anwesenheit von Kaliumhydrid ist.

7. Verfahren nach irgendeinem der vorstehenden Ansprüche, **dadurch gekennzeichnet, daß** die Reaktion in einem inerten organischen Medium unter den Bedingungen der Reaktion realisiert wird.

8. Verfahren nach Anspruch 4 bis 6, **dadurch gekennzeichnet, daß** man ein unter den Ethern ausgewähltes Lösungsmittel verwendet.

9. Verfahren nach Anspruch 3, **dadurch gekennzeichnet, daß** in dem Fall, wo die Reaktion in Anwesenheit von Silberoxid realisiert wird, das verwendete Lösungsmittel unter den aromatischen Lösungsmitteln und den polaren aprotischen Lösungsmitteln ausgewählt wird.

10. Verfahren nach Anspruch 8, **dadurch gekennzeichnet, daß** man als Lösungsmittel Tetrahydrofuran oder Dimethoxyethan verwendet.

11. Verfahren nach Anspruch 1 und 3, **dadurch gekennzeichnet, daß** man eine Mischung von 1,8-Bis-(Dimethylamino)-naphthalin und Trimethyloxonium-tetrafluorborat in Ethylacetat verwendet.

12. Verfahren nach irgendeinem der vorstehenden Ansprüche, **dadurch gekennzeichnet, daß** man ein molares Verhältnis zwischen dem Anionisierungsmittel und dem Substrat von über 2 und vorzugsweise zwischen 2 und 20 verwendet.

13. Verfahren nach irgendeinem der vorstehenden Ansprüche, **dadurch gekennzeichnet, daß** man ein molares Verhältnis zwischen dem Alkylierungsmittel und dem Substrat von über 2 und vorzugsweise zwischen 2 und 40 verwendet.

14. Verfahren nach irgendeinem der vorstehenden Ansprüche, **dadurch gekennzeichnet, daß** man eine Reaktionstemperatur zwischen -30 °C und 80 °C verwendet.

## Claims

1. Process for the one-step direct alkylation, in positions 7 and 10 simultaneously of the two hydroxyl functions on 10-deacetylbaccatin or on derivatives thereof which are esterified in position 13, of formula (V) 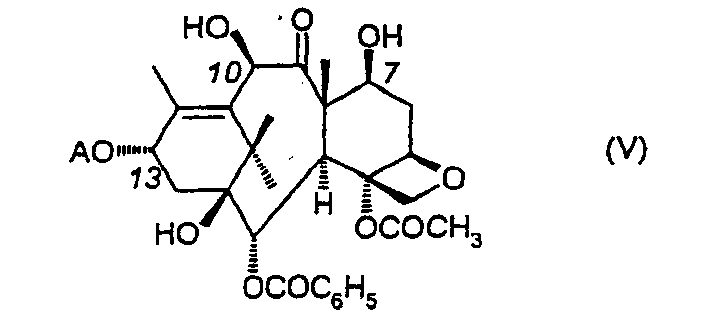 in which A represents hydrogen or a side chain of formula (Ic) below: 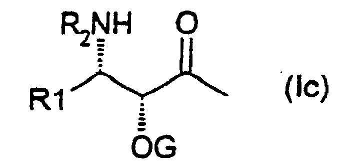
• G represents a protecting group for the hydroxyl function,
• R₁ represents
1) a straight or branched alkyl radical containing 1 to 8 carbon atoms, a straight or branched alkenyl radical containing 2 to 8 carbon atoms, a straight or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or
2) a 5-membered aromatic heterocycle containing one or more hetero atoms, which may be identical or different, chosen from nitrogen, oxygen and sulphur atoms, and optionally substituted with one or more substituents, which may be identical or different, chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl, and alkoxycarbonyl radicals,
3) it being understood that, in the substituents on the phenyl, α- or β-naphthyl radicals and the aromatic heterocycles, the alkyl radicals and the alkyl portions of the other radicals contain 1 to 4 carbon atoms and that the alkenyl and alkynyl radicals contain 2 to 8 carbon atoms and that the aryl radicals are phenyl or α- or β-naphthyl radicals,
• R₂ represents
1) a benzoyl radical optionally substituted with one or more atoms or radicals, which may be identical or different, chosen from halogen atoms and alkyl radicals containing 1 to 4 carbon atoms, alkoxy radicals containing 1 to 4 carbon atoms or trifluoromethyl, thenoyl or furoyl radicals, or
2) a radical R'₂-O-CO- in which R'₂ represents:
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals optionally being substituted with one or more substituents chosen from halogen atoms and hydroxyl radicals, alkoxy radicals containing 1 to 4 carbon atoms, dialkylamino radicals in which each alkyl part contains 1 to 4 carbon atoms, piperidino or morpholino radicals, 1-piperazinyl radicals (optionally substituted in position 4 with an alkyl radical containing 1 to 4 carbon atoms or with a phenyl alkyl radical in which the alkyl part contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl part contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms or a 5-membered aromatic heterocyclic radical preferably chosen from furyl and thienyl radicals,
or a saturated heterocyclic radical containing 4 to 6 carbon atoms, optionally substituted with one or more alkyl radicals containing 1 to 4 carbon atoms,
or an oxazolidine of formula (Id) below: 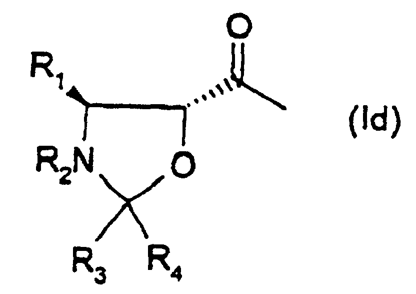 in which R₃ and R₄ are chosen from hydrogen or alkyl, aryl, halo, alkoxy, arylalkyl, alkoxyaryl, haloalkyl or haloaryl radicals, it being possible for the substituents optionally to form a 4- to 7-membered ring,
**characterized in that** the alkylating agent is chosen from:
• alkyl halides and preferably, among those, alkyl iodides (RI)
• alkyl sulphates such as methyl sulphate
• oxoniums such as boric salts of trialkyloxoniums, in particular trimethyloxonium tetrafluoroborate
in the presence of one or more anionization agents such as one or more strong bases in anhydrous medium.

2. Process according to claim 1, **characterized in that** the alkylating agent is methyl sulphate or iodide and preferably methyl iodide.

3. Process according to claim 1, **characterized in that** the bases which can be used in anhydrous medium are chosen from alkali metal hydrides, alkali metal alkoxides, silver oxide, 1,8-bis(dimethylamino)naphthalene, alkali metal amides mixed with alkali metal t-butoxides or alkyllithiums mixed with alkali metal t-butoxides.

4. Process according to claim 3, **characterized in that** the alkali metal hydrides are chosen from sodium or potassium hydride and preferably potassium hydride.

5. Process according to claim 3, **characterized in that** the alkali metal alkoxide is potassium tert-butoxide.

6. Process according to claim 1, **characterized in that** the alkylating agent is methyl iodide in the presence of potassium hydride.

7. Process according to any one of the preceding claims, **characterized in that** the reaction is carried out in an organic medium which is inert under the reaction conditions.

8. Process according to claims 4 to 6, **characterized in that** a solvent chosen from ethers is used.

9. Process according to claim 3, **characterized in that** when the reaction is carried out in the presence of silver oxide, the solvent used is chosen from aromatic solvents and polar aprotic solvents.

10. Process according to claim 8, **characterized in that** tetrahydrofuran or dimethoxyethane is used as solvent.

11. Process according to claims 1 and 3, **characterized in that** a mixture of 1,8-bis(dimethylamino)-naphthalene and trimethyloxonium tetrafluoroborate in ethyl acetate is used.

12. Process according to any one of the preceding claims, **characterized in that** a molar ratio between the anionization agent and the substrate of greater than 2 and preferably between 2 and 20 is used.

13. Process according to any one of the preceding claims, **characterized in that** a molar ratio between the alkylating agent and the substrate of greater than 2 and preferably between 2 and 40 is used.

14. Process according to any one of the preceding claims, **characterized in that** a reaction temperature of between -30°C and 80°C is used.
